# EUROPEAN PATENT APPLICATION

(11) **EP 4 759 255 A1**
(43) Date of publication of application: **17.06.2026**
(21) Application number: 24934646.1
(22) Date of filing: 17.06.2024
(51) Int. Cl.: A61B 18/00

(54) **ENERGY-BASED SURGERY ACTIVE COOLING APPARATUS AND ENERGY-BASED SURGERY ACTIVE COOLING SYSTEM**

(30) Priority: 12.04.2024 CN 202410443428
(71) Applicant: Nanchang Huaan Zhonghui Health Technology Co., Ltd., Nanchang, Jiangxi 330095 (CN)
(72) Inventor: FAN, Xiaolei, Nanchang, Jiangxi 330095 (CN)
(74) Representative: Lavoix
(86) International application number: PCT/CN2024/099574
(87) International publication number: WO 2025/213576

(57) **Abstract**

The present application relates to the technical field of medical instruments and discloses an energy-based surgery active cooling apparatus and an energy-based surgery active cooling system. The energy-based surgery active cooling apparatus comprises: a first clamping body; a second clamping body connected to the first clamping body, wherein the far end of the second clamping body is suitable for abutting against the far end of the first clamping body; and a cooling structure arranged on at least one of the first clamping body and the second clamping body. The cooling structure is hollow and forms an accommodating cavity suitable for accommodating a cooling medium. The accommodating cavity is in communication with an external cold source and is suitable for circulating the cooling medium with the external cold source to conduct the heat on the first clamping body and/or the second clamping body from a far-end tip to the external cold source. The energy-based surgery active cooling apparatus provided by the present application not only eliminates the heat risk of the heating surface and improves the heat dissipation and cooling efficiency of the clamping bodies, but also simplifies the heat dissipation structure of the clamping bodies, thereby simplifying the process for the apparatus.

## Description

### CROSS-REFERENCE TO RELATED APPLICATIONS

The present application claims the priority of the Chinese patent application with the application number of 202410443428.7, filed with the CNIPA on April 12, 2024 and entitled "Energy-Based Surgery Active Cooling Apparatus and Energy-Based Surgery Active Cooling System", which is incorporated herein by reference in its entirety.

### TECHNICAL FIELD

The present application relates to the technical field of medical instruments, and more particularly, to an energy-based surgery active cooling apparatus and an energy-based surgery active cooling system.

### BACKGROUND

Energy medical instruments are mainly used in surgical scenarios such as tissue cutting and vascular or tissue anastomosis sealing in surgical operations. Currently, a critical issue to be addressed in clinical surgery is the thermal diffusion injury to adjacent or surrounding tissues, which is caused by the heat diffusion of the electrode at the sealed site.

Existing energy surgical electrode assemblies typically utilize the principle of thermoelectric cooling plates, a thermoelectric cooling plate is designed in the forceps head area, and the direction of cooling-heating conversion is controlled to achieve active cooling and heat dissipation in the clamping direction of the forceps. Therefore, the cooling efficiency of the forceps head is relatively low.

### SUMMARY OF THE INVENTION

In view of this, the present application provides an energy-based surgery active cooling apparatus and an energy-based surgery active cooling system to solve the problem of low cooling efficiency of the forceps heads of existing energy-based surgery electrode assemblies.

In a first aspect, the present application provides an energy-based surgery active cooling apparatus, including:
a first clamping body;
a second clamping body connected to the first clamping body, wherein a distal end of the second clamping body is adapted to abut against a distal end of the first clamping body;
a cooling structure disposed on at least one of the first clamping body and the second clamping body; wherein the cooling structure is hollow inside and to form an accommodating cavity, which is adapted to accommodate a cooling medium;
the accommodating cavity is in communication with an external cold source, and the accommodating cavity is adapted to circulate the cooling medium with the external cold source, so as to conduct the heat on the first clamping body and/or the second clamping body from the distal tip to the external cold source.

Beneficial effects: The energy-based surgery active cooling apparatus provided by the present application realizes cooling and temperature reduction of the heat-generating parts of the first clamping body and/or the second clamping body by arranging a cooling structure on at least one of the first clamping body and the second clamping body, where the cooling structure is hollow inside to form an accommodating cavity for accommodating a cooling medium. The accommodating cavity is in communication with an external cold source, so that the heat of the heat-generating parts is continuously transferred to the external cold source by the circulation of the cooling medium. This not only eliminates the heat risk of the heat-generating surface, avoids the need to arrange another set of heat dissipation structures in the clamping body for synchronous heat conduction, improves the heat dissipation and cooling efficiency of the clamping body, but also simplifies the heat dissipation structures of the clamping body and reduces the process difficulty of the energy-based surgery active cooling apparatus.

In an optional embodiment, the first clamping body includes a forceps body and a forceps tip; the cooling structure includes a cooling block body and a first sealing head;
the cooling block body is independently disposed at a tip position of the forceps tip of the first clamping body, the cooling block body is disposed on a side of the first clamping body away from the second clamping body along a first direction, and the cooling block body is attached to the side of the first clamping body away from the second clamping body along the first direction;
a first accommodating groove is formed on the cooling block body; the first sealing head is adapted to seal the first accommodating groove to close the first accommodating groove and form the accommodating cavity.

Beneficial effects: The cooling block body is attached to the side of the first clamping body away from the second clamping body along the first direction, so as to ensure the heat transfer area between the cooling block body and the first clamping body, improve the heat transfer efficiency between the cooling block body and the first clamping body, facilitate the cooling block body to timely take away the heat on the clamping body, help improve the cooling efficiency, and avoid redundant heat from damaging surrounding blood vessels or nerves. The first sealing head seals the first accommodating groove to close the first accommodating groove and form the accommodating cavity, thereby reducing the processing difficulty of the first accommodating groove and the assembly difficulty of the cooling structure.

In an optional embodiment, the cooling structure further includes a first tube body and a second tube body, both of which are disposed on the side of the first clamping body away from the second clamping body along the first direction;
a distal end of the first tube body is in communication with the accommodating cavity, a proximal end of the first tube body is adapted to be in communication with the external cold source, and the first tube body is adapted to continuously introduce the cooling medium from the external cold source into the accommodating cavity;
a distal end of the second tube body is in communication with the accommodating cavity, a proximal end of the second tube body is adapted to be in communication with the external cold source, and the second tube body is adapted to continuously guide the cooling medium after heat absorption from the accommodating cavity to the external cold source.

Beneficial effects: The first tube body and the second tube body are disposed to form a circulating cooling loop between the cooling block body and the external cold source, which not only prevents the generation of excess heat, thereby avoiding redundant heat from damaging surrounding blood vessels or nerves, but also can greatly improve the cooling efficiency of the cooling structure for the first clamping body.

In an optional embodiment, the first clamping body includes a forceps body and a forceps tip; the cooling structure includes a third tube body, which is disposed on a side of the first clamping body away from the second clamping body along a first direction; the third tube body is of a U-shaped structure and is bent to change direction at a distal end position of the forceps tip of the first clamping body;
the third tube body is of an integrally formed structure, an input end of the third tube body is in communication with a cooling medium output port of the external cold source, an output end of the third tube body is in communication with a cooling medium return port of the external cold source, and the third tube body is adapted to circulate the cooling medium to cool the first clamping body.

Beneficial effects: During operation, the third tube body can timely take away the heat on both sides of the first clamping body along the first direction without generating excess heat, so that a low-temperature safety zone is formed within the entire circumferential area of the first clamping body, thereby effectively avoiding damage to surrounding blood vessels or nerves and greatly improving the safety of electrosurgery. On the other hand, the third tube body is of an integrally formed U-shaped structure, which does not require additional heat dissipation components and sealing components, thereby greatly simplifying the cooling structure and reducing the process difficulty.

In an optional embodiment, the first clamping body includes a forceps body and a forceps tip; the cooling structure includes a fourth tube body and a circulating cooling assembly, both of which are disposed on a side of the first clamping body away from the second clamping body along a first direction;
the fourth tube body is disposed at an end of the first clamping body close to the forceps tip along an axial direction, and the fourth tube body is adapted to accommodate the cooling medium to cool and reduce the temperature of the first clamping body;
the circulating cooling assembly is disposed at an end of the first clamping body close to the forceps body along the axial direction, a distal end of the circulating cooling assembly is in communication with the fourth tube body, a proximal end of the circulating cooling assembly is adapted to be in communication with the external cold source, and the circulating cooling assembly is adapted to continuously cool the cooling medium in the fourth tube body.

Beneficial effects: During operation, a circulating loop is formed between the circulating cooling assembly and the external cold source, and at the same time, heat is transferred between the circulating cooling assembly and the fourth tube body, so as to conduct the heat on the first clamping body to the external cold source, thereby realizing cooling and temperature reduction of the first clamping body. The fourth tube body is disposed at an end of the first clamping body close to the forceps tip along an axial direction, which not only can realize cooling and temperature reduction of the first clamping body, but also can reduce the thickness of the forceps tip position of the first clamping body along a first direction and the width along a second direction, thereby reducing the volume of the forceps tip. On the premise of ensuring the basic clamping and cutting functions as well as cooling efficiency, the first clamping body can be more miniaturized, so as to avoid the forceps head touching nerves or blood vessels in a complex and narrow surgical space, avoid heat damage risks, and greatly improve the safety of electrosurgery.

In an optional embodiment, the circulating cooling assembly includes a communicating block, a fifth tube body and a sixth tube body, and the communicating block is adapted to simultaneously communicate the fourth tube body, the fifth tube body and the sixth tube body;
both the fifth tube body and the sixth tube body are disposed at a proximal end of the first clamping body; a distal end of the fifth tube body is in communication with the communicating block, a proximal end of the fifth tube body is adapted to be in communication with a cooling medium output port of the external cold source; a distal end of the sixth tube body is in communication with the communicating block, and a proximal end of the sixth tube body is adapted to be in communication with a cooling medium return port of the external cold source.

Beneficial effects: The communicating block is disposed to simultaneously communicate the fourth tube body, the fifth tube body and the sixth tube body, so that the heat in the fourth tube body can be conducted into the communicating block, and continuous circulating cooling is performed through the fifth tube body and the sixth tube body, so as to conduct the heat in the fourth tube body to the external cold source. This allows the fourth tube body to timely take away the heat of the first clamping body without generating excess heat, which effectively avoids damage to surrounding blood vessels or nerves, and improves the safety of electrosurgery. At the same time, the first clamping body can be more miniaturized, so as to avoid the forceps head touching nerves or blood vessels in a complex and narrow surgical space, avoid heat damage risks, and greatly improve the safety of electrosurgery.

In an optional embodiment, the first clamping body includes a forceps body and a forceps tip; the cooling structure includes a first plate body and a second sealing head;
the first plate body is fixedly disposed on a side of the first clamping body close to the second clamping body along a first direction, and the first plate body is adapted to abut against the second clamping body; a cooling portion is formed on a side of the first plate body away from the second clamping body along the first direction, the cooling portion is disposed at an end of the first plate body close to the forceps tip along an axial direction, and the cooling portion and the first plate body are of an integrally formed structure;
a second accommodating groove is formed on the cooling portion; the second sealing head is adapted to seal the second accommodating groove to close the second accommodating groove and form the accommodating cavity.

Beneficial effects: The part to be cooled of the first clamping body is disassembled into a single component to be directly connected to the waterway, avoiding the need to arrange an independent cooling block, thereby further improving the cooling and heat dissipation efficiency.

In an optional embodiment, the cooling structure further includes a seventh tube body and an eighth tube body, both of which are disposed on a side of the first clamping body away from the second clamping body along the first direction;
a distal end of the seventh tube body is in communication with the accommodating cavity, a proximal end of the seventh tube body is adapted to be in communication with the external cold source, and the seventh tube body is adapted to continuously introduce the cooling medium from the external cold source into the accommodating cavity;
a distal end of the eighth tube body is in communication with the accommodating cavity, a proximal end of the eighth tube body is adapted to be in communication with the external cold source, and the eighth tube body is adapted to continuously guide the cooling medium after heat absorption from the accommodating cavity to the external cold source.

Beneficial effects: The seventh tube body and the eighth tube body are disposed to form a circulating cooling loop between the cooling portion of the first plate body and the external cold source, which not only prevents the generation of excess heat, avoids redundant heat from damaging surrounding blood vessels or nerves, but also can greatly improve the cooling efficiency of the cooling structure for the first plate body.

In an optional embodiment, the first clamping body includes a forceps body and a forceps tip; the cooling structure includes a housing portion, which is disposed at an end of the first clamping body close to the forceps tip along an axial direction, and the housing portion and the first clamping body are integrally formed;
a third accommodating groove is formed on a side of the housing portion close to the second clamping body along a first direction; the cooling structure further includes a second plate body, which is fixedly disposed on a side of the first clamping body close to the second clamping body along the first direction, and the second plate body is adapted to cover the third accommodating groove to close the third accommodating groove and form the accommodating cavity.

Beneficial effects: The accommodating cavity has a larger volume and can accommodate more cooling medium, which greatly increases the cooling area for the first clamping body and is conducive to improving the cooling efficiency of the first clamping body.

In an optional embodiment, a first through hole and a second through hole are formed at a proximal end of the first clamping body, and the first through hole and the second through hole respectively extend axially toward the forceps tip and are both in communication with the third accommodating groove;
the cooling structure further includes a ninth tube body and a tenth tube body, both of which are disposed at an end of the first clamping body away from the forceps tip along the axial direction;
a distal end of the ninth tube body is in communication with the first through hole, a proximal end of the ninth tube body is adapted to be in communication with the external cold source, and the ninth tube body is adapted to continuously introduce the cooling medium from the external cold source into the accommodating cavity via the first through hole;
a distal end of the tenth tube body is in communication with the second through hole, a proximal end of the tenth tube body is adapted to be in communication with the external cold source, and the tenth tube body is adapted to continuously guide the cooling medium after heat absorption from the accommodating cavity to the external cold source via the second through hole.

Beneficial effects: The first through hole, the ninth tube body, the second through hole and the tenth tube body are disposed to form a circulating cooling loop between the accommodating cavity enclosed by the housing portion and the second plate body and the external cold source, which not only prevents the generation of excess heat, avoids redundant heat from damaging surrounding blood vessels or nerves, but also can greatly improve the cooling efficiency of the cooling structure for the first clamping body.

In an optional embodiment, the first clamping body includes a tweezer body and a tweezer tip; a tip position of the tweezer tip of the first clamping body is hollow inside and to form the accommodating cavity;
the cooling structure includes an eleventh tube body and a twelfth tube body, which are respectively disposed on both sides of the tweezer body of the first clamping body along a second direction and extend axially to the tip position of the tweezer tip of the first clamping body;
a distal end of the eleventh tube body is in communication with the accommodating cavity, a proximal end of the eleventh tube body is in communication with a cooling medium output port of the external cold source, and the eleventh tube body is adapted to continuously introduce the cooling medium from the external cold source into the accommodating cavity; a distal end of the twelfth tube body is in communication with the accommodating cavity, a proximal end of the twelfth tube body is in communication with a cooling medium return port of the external cold source, and the twelfth tube body is adapted to continuously guide the cooling medium after heat absorption from the accommodating cavity to the external cold source.

Beneficial effects: The eleventh tube body and the twelfth tube body are disposed to form a circulating cooling loop between the accommodating cavity at the tip position of the forceps tip of the first clamping body and the external cold source. During operation, the cooling structure and the cooling medium therein can timely take away the heat at the tip of the forceps tip without generating excess heat, so that a low-temperature safety zone is formed within the entire circumferential area of the first clamping body and/or the second clamping body. This facilitates an operator or a physician to selectively use the first clamping body as a fulcrum away from nerves or blood vessels in the actual clinical surgical process, thereby effectively avoiding damage to surrounding blood vessels or nerves caused by redundant heat generated during electrocoagulation or electrocision due to heating of both clamping bodies, and greatly improving the safety of electrosurgery. At the same time, it eliminates the heat risk of the heat-generating surface, and avoids the need to arrange another set of heat dissipation structures in the clamping body for synchronous heat conduction, which not only improves the heat dissipation and cooling efficiency of the clamping body, enhances the safety in the clinical surgical process, but also simplifies the heat dissipation structures of the clamping body and reduces the process difficulty of the energy-based surgery active cooling apparatus.

In an optional embodiment, the second clamping body includes a tweezer body and a tweezer tip; the tip position of the tweezer tip of the second clamping body is hollow inside and to form the accommodating cavity;
the cooling structure includes a thirteenth tube body and a fourteenth tube body, which are respectively disposed on both sides of the tweezer body of the second clamping body along the second direction and extend axially to the tip position of the tweezer tip of the second clamping body;
a distal end of the thirteenth tube body is in communication with the accommodating cavity, a proximal end of the thirteenth tube body is in communication with the cooling medium output port of the external cold source, and the thirteenth tube body is adapted to continuously introduce the cooling medium from the external cold source into the accommodating cavity; a distal end of the fourteenth tube body is in communication with the accommodating cavity, a proximal end of the fourteenth tube body is in communication with the cooling medium return port of the external cold source, and the fourteenth tube body is adapted to continuously guide the cooling medium after heat absorption from the accommodating cavity to the external cold source.

Beneficial effects: During operation, the cooling structure of the "double-side forceps body cooling structure" and the cooling medium therein can timely take away the heat at the tips of the forceps tips of both clamping bodies without generating excess heat, so that a low-temperature safety zone is formed within the entire circumferential area of the first clamping body and the second clamping body, thereby further improving the cooling and heat dissipation efficiency.

In an optional embodiment, the energy-based surgery active cooling apparatus further includes a connecting block, which is disposed between the first clamping body and the second clamping body, and the connecting block is adapted to connect a proximal end of the first clamping body with a proximal end of the second clamping body.

Beneficial effects: In the energy-based surgery active cooling apparatus applied to monopolar and bipolar metal electrodes under laparoscopy, the connecting block is adapted to rotatably connect the first clamping body and the second clamping body to realize the basic clamping and opening function of the forceps. In the energy-based surgery active cooling apparatus applied to high-frequency bipolar tweezer, the connecting block is adapted to fixedly connect the first clamping body and the second clamping body to realize the basic forceps tip clamping function.

In a second aspect, the present application further provides an energy-based surgery active cooling system, including: an external cold source and the energy-based surgery active cooling apparatus as described above, wherein the external cold source is adapted to supply the cooling medium to the cooling structure.

Beneficial effects: The energy-based surgery active cooling system of the second aspect includes the energy-based surgery active cooling apparatus of the first aspect, therefore, the energy-based surgery active cooling system of the second aspect includes all the beneficial effects of the energy-based surgery active cooling apparatus of the first aspect.

In an optional embodiment, the energy-based surgery active cooling system further includes a pumping device, which is adapted to provide power for the circulation of the cooling medium between the cooling structure and the external cold source.

Beneficial effects: The energy-based surgery active cooling system is provided with a pumping device to provide power for the circulation of the cooling medium between the cooling structure and the external cold source. During operation, the pumping device can be used to adjust the circulation flow rate and flow volume of the cooling medium, thereby adjusting the cooling and temperature reduction effect of the cooling structure for the clamping body.

In an optional embodiment, the energy-based surgery active cooling system further includes a refrigeration device, which is adapted to cool the cooling medium.

Beneficial effects: The energy-based surgery active cooling system is provided with a refrigeration device to cool the cooling medium, thereby increasing the temperature difference between the heating part of the clamping body and the cooling medium, and improving the cooling and temperature reduction efficiency of the cooling structure for the clamping body.

### BRIEF DESCRIPTION OF THE DRAWINGS

In order to more clearly explain technical solutions in specific embodiments of the present application or in the prior art, the accompanying drawings that need to be used in the description of the specific embodiments or the prior art will be briefly introduced below. Apparently, the accompanying drawings described below only represent some embodiments of the present application. For those ordinarily skilled in the art, other drawings may also be obtained based on these accompanying drawings without expenditure of creative efforts.
FIG. 1 is a perspective structural schematic view of an energy-based surgery active cooling apparatus according to the first embodiment of the present application;
FIG. 2 is a working principle view of an energy-based surgery active cooling apparatus according to the first embodiment of the present application;
FIG. 3 is an exploded structural schematic view of an energy-based surgery active cooling apparatus according to the first embodiment of the present application;
FIG. 4 is a working principle view of an energy-based surgery active cooling apparatus according to the second embodiment of the present application;
FIG. 5 is an exploded structural schematic view of an energy-based surgery active cooling apparatus according to the second embodiment of the present application;
FIG. 6 is a working principle view of an energy-based surgery active cooling apparatus according to the third embodiment of the present application;
FIG. 7 is an exploded structural schematic view of an energy-based surgery active cooling apparatus according to the third embodiment of the present application;
FIG. 8 is a working principle view of an energy-based surgery active cooling apparatus according to the fourth embodiment of the present application;
FIG. 9 is an exploded structural schematic view of an energy-based surgery active cooling apparatus according to the fourth embodiment of the present application;
FIG. 10 is a working principle view of an energy-based surgery active cooling apparatus according to the fifth embodiment of the present application;
FIG. 11 is an exploded structural schematic view of an energy-based surgery active cooling apparatus according to the fifth embodiment of the present application;
FIG. 12 is a perspective structural schematic view of an energy-based surgery active cooling apparatus according to the sixth embodiment of the present application;
FIG. 13 is an exploded structural schematic view of an energy-based surgery active cooling apparatus according to the sixth embodiment of the present application;
FIG. 14 is an exploded structural schematic view of an energy-based surgery active cooling apparatus according to the seventh embodiment of the present application;
FIG. 15 is a partial cross-sectional enlarged schematic view of position Q in FIG. 12;
FIG. 16 is a working principle schematic view of an energy-based surgery active cooling system according to the eighth embodiment of the present application; and
FIG. 17 is a working principle schematic view of an energy-based surgery active cooling system according to the ninth embodiment of the present application.

List of Reference Signs:
101. First clamping body, 102. Second clamping body, 103. Cooling structure, 104. Accommodating cavity, 105. Connecting block, 106. Forceps body, 107. Forceps tip, 108. Tweezer body, 109. Tweezer tip;
200. External cold source, 300. Pumping device, 400. Refrigeration device;
11. Cooling block body, 111. First accommodating groove, 12. First sealing head, 13. First tube body, 14. Second tube body;
21. Third tube body;
31. Fourth tube body, 32. Circulating cooling assembly, 321. Communicating block, 322. Fifth tube body, 323. Sixth tube body;
41. First plate body, 411. Cooling portion, 412. Second accommodating groove, 42. Second sealing head, 43. Seventh tube body, 44. Eighth tube body;
51. Housing portion, 511. Third accommodating groove, 52. Second plate body, 53. First through hole, 54. Second through hole, 55. Ninth tube body, 56. Tenth tube body;
61. Eleventh tube body; 62. Twelfth tube body;
71. Thirteenth tube body; 72. Fourteenth tube body.

### DETAILED DESCRIPTION OF THE EMBODIMENTS

The energy-based surgical electrode assemblies in the related technology have thermoelectric cooling plates designed in the forceps head area based on the principle of thermoelectric cooling plates, and realizes cooling and heat dissipation in the clamping direction of the forceps head by controlling the direction of cold-heat conversion. However, on the one hand, the basic principle of thermoelectric cooling plates is an energy conversion technology that utilizes the Peltier effect of semiconductor materials to achieve refrigeration or heating. When a DC power supply is connected, the temperature of one end of the thermoelectric refrigeration device will decrease, while the temperature of the other end will increase at the same time, which not only has a low refrigeration performance coefficient, but also generates new heat on the other side while refrigerating one side due to the principle. This part of heat needs another set of heat dissipation mechanism for synchronous heat conduction, which not only has a complex structure and high process difficulty, but also has low cooling efficiency of the forceps head. On the other hand, the existing energy-based surgical electrode assemblies are only applicable to clinical surgical scenarios where the fascia around blood vessels has been dissected. In such surgical scenarios, the surgical space is relatively large, thereby allowing the heat-generating area on the back of the electrothermal refrigeration device to have space for dissipate heat through natural convection or other means, but the heat risk of the heat-generating surface still exists essentially. More importantly, in actual clinical surgical processes such as tumor resection and fascia dissection, an operator is actually operating in a very narrow space, and surrounding blood vessels and nerves are always close to the forceps head of the instrument. When both forceps heads of the bipolar electrode are heated, if a physician or an operator touches a nerve or blood vessel with either forceps head due to unavoidable factors such as fatigue, it will cause irreparable trauma, which has great safety risks.

In order to make the purposes, technical solutions and advantages of the embodiments of the present application clearer, the technical solutions in the embodiments of the present application will be clearly and completely described below in conjunction with the accompanying drawings in the embodiments of the present application. Obviously, the described embodiments are part of the embodiments of the present application, rather than all the embodiments. Based on the embodiments in the present application, all other embodiments obtained by those skilled in the art without expenditure of creative efforts shall fall within the protection scope of the present application.

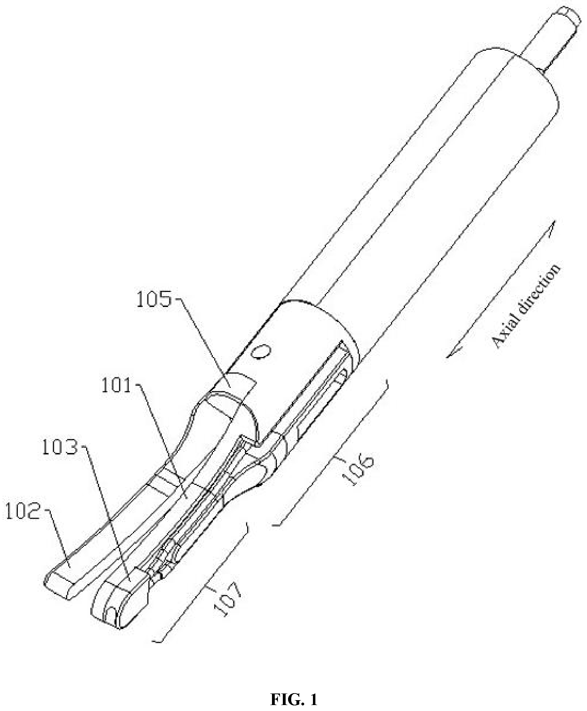

The embodiments of the present application will be described below in conjunction with FIGS. 1 to 17.

According to the embodiments of the present application, in one aspect, an energy-based surgery active cooling apparatus is provided, including:
a first clamping body 101;
a second clamping body 102, connected to the first clamping body 101, wherein a distal end of the second clamping body 102 is adapted to abut against a distal end of the first clamping body 101;
a cooling structure 103, disposed on at least one of the first clamping body 101 and the second clamping body 102; wherein the cooling structure 103 is hollow inside and to form an accommodating cavity 104, which is adapted to accommodate a cooling medium;
the accommodating cavity 104 is in communication with an external cold source 200, and the accommodating cavity 104 is adapted to circulate the cooling medium with the external cold source 200, so as to conduct the heat on the first clamping body 101 and/or the second clamping body 102 from the distal tip to the external cold source 200.

It should be noted that for better understanding, the terms "proximal (end/side)" and "distal (end/side)" are defined from the perspective of the doctor (or other surgeon). Therefore, the term "proximal (end/side)" is used to indicate a side or an end or the part thereon of the device closest to the external body wall and/or the surgeon, while the term "distal (end/side)" refers to the structural side or end in the direction opposite to the external body wall and/or the surgeon.

It should be noted that the "cooling medium" has thermal conductivity, which can be a gaseous cooling medium, a liquid cooling medium, or a gas-liquid mixed cooling medium. The specific composition of the cooling medium can be configured according to actual clinical needs, which is not specifically limited herein.

It is worth noting that compared with the heat dissipation method of the electrode sealing assembly in the related technology, the energy-based surgery active cooling apparatus provided by the present application realizes continuous cooling circulation between the cooling structure 103 and the external cold source 200, so that the cooling medium in the accommodating cavity 104 is always in a good low-temperature cooling state. During operation, the cooling structure 103 and the cooling medium therein can not only timely take away the heat on the side of the first clamping body 101 facing the second clamping body 102, but also timely take away the heat on the side of the first clamping body 101 away from the second clamping body 102 without generating excess heat, so that a low-temperature safety zone is formed within the entire circumferential area of the first clamping body 101 and/or the second clamping body 102, thereby facilitating the operator or physician to selectively use the first clamping body 101 and/or the second clamping body 102 as a fulcrum away from nerves or blood vessels in the actual clinical surgical process, effectively avoiding damage to surrounding blood vessels or nerves caused by redundant heat generated during electrocoagulation or electrocision due to heating of both clamping bodies, and greatly improving the safety of electrosurgery. Compared with the heat dissipation structure of the electrode sealing assembly in the related technology, the energy-based surgery active cooling apparatus provided by the present application realizes continuous cooling circulation between the cooling structure 103 and the external cold source 200 by arranging the cooling structure 103 to communicate the accommodating cavity 104 of the cooling structure 103 with the external cold source 200, thereby eliminating the heat risk of the heat-generating surface, and avoiding the need to arrange another set of heat dissipation structures in the clamping body for synchronous heat conduction. This not only improves the heat dissipation and cooling efficiency of the clamping body, enhances the safety in the clinical surgical process, but also simplifies the heat dissipation structure of the clamping body and reduces the process difficulty of the energy-based surgery active cooling apparatus.

The energy-based surgery active cooling apparatus provided by the present application realizes cooling and temperature reduction of the heat-generating parts of the first clamping body 101 and/or the second clamping body 102 by arranging a cooling structure 103 on at least one of the first clamping body 101 and the second clamping body 102, where the cooling structure 103 is hollow inside to form an accommodating cavity 104 for accommodating a cooling medium. The accommodating cavity 104 is in communication with an external cold source 200, so that the heat of the heat-generating parts is continuously transferred to the external cold source 200 through the circulation of the cooling medium. This not only eliminates the heat risk of the heat-generating surface, avoids the need to arrange another set of heat dissipation structures in the clamping body for synchronous heat conduction, improves the heat dissipation and cooling efficiency of the clamping body, but also simplifies the heat dissipation structure of the clamping body and reduces the process difficulty of the energy-based surgery active cooling apparatus.

It should be noted that the energy-based surgery active cooling apparatus provided by the present application is mainly used for intraoperative active cooling of energy instruments in electrosurgery. In order to facilitate a better explanation and understanding of the present application, the following description will be given by taking the energy-based surgery active cooling apparatus applied to monopolar and bipolar metal electrodes under laparoscopy and the energy-based surgery active cooling apparatus applied to high-frequency bipolar tweezers as examples respectively.

Referring to FIGS. 1 to 3 together, the following exemplarily provides a first type of energy-based surgery active cooling apparatus applied to monopolar and bipolar metal electrodes under laparoscopy, wherein the cooling structure 103 mainly includes a cooling block body 11, a first sealing head 12, a first tube body 13 and a second tube body 14, thereby forming an "independent cooling block-independent double-tube body cooling structure".

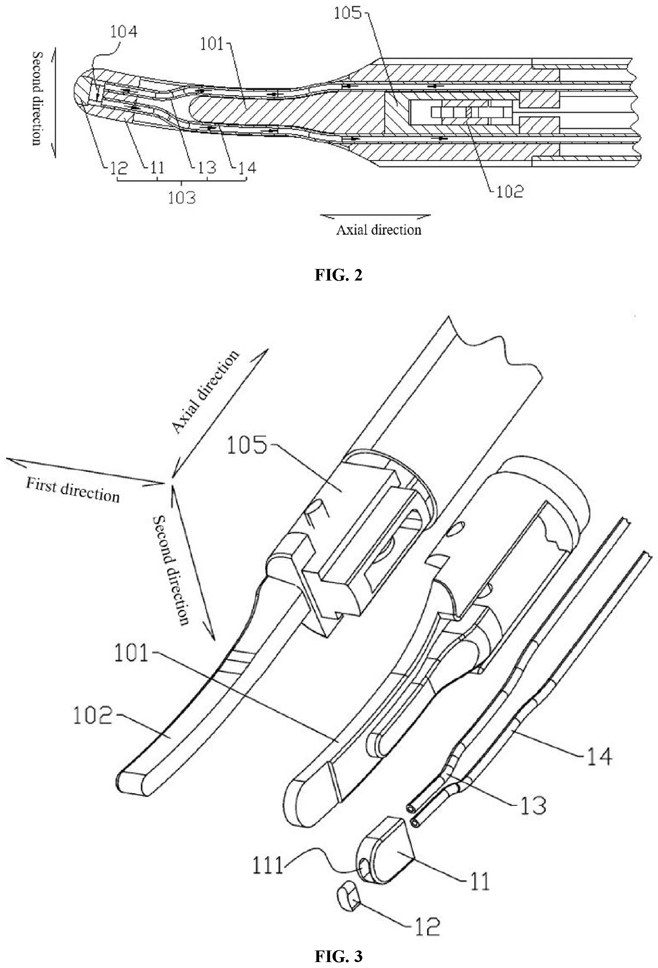

In some embodiments, referring to FIG. 1, the first clamping body 101 includes a forceps body 106 and a forceps tip 107, referring to FIG. 2, the cooling structure 103 includes a cooling block body 11 and a first sealing head 12;
referring to FIG. 3 together, the cooling block body 11 is independently disposed at the tip position of the forceps tip 107 of the first clamping body 101, the cooling block body 11 is fixedly disposed on a side of the first clamping body 101 away from the second clamping body 102 along an first direction, and the cooling block body 11 is attached to the side of the first clamping body 101 away from the second clamping body 102 along the first direction, so as to ensure the heat transfer area between the cooling block body 11 and the first clamping body 101, improve the heat transfer efficiency between the cooling block body 11 and the first clamping body 101, facilitate the cooling block body 11 to timely take away the heat on the clamping body, help improve the cooling efficiency, and avoid redundant heat from damaging surrounding blood vessels or nerves;
a first accommodating groove 111 is formed on the cooling block body 11, the first sealing head 12 is adapted to seal the first accommodating groove 111 to close the first accommodating groove 111 and form the accommodating cavity 104, thereby reducing the processing difficulty of the first accommodating groove 111 and the assembly difficulty of the cooling structure 103.

Optionally, referring to FIG. 3, a recessed platform (not shown in the figure) may be formed at the tip position of the forceps tip 107 of the first clamping body 101, and the recessed platform is adapted to be attached to and fixedly connected to one side of the cooling block body 11.

In some embodiments, referring to FIG. 3, the cooling structure 103 further includes a first tube body 13 and a second tube body 14, both of which are independently disposed on the side of the first clamping body 101 away from the second clamping body 102 along the first direction;

Referring to FIG. 2 together, a distal end of the first tube body 13 is in communication with the accommodating cavity 104, a proximal end of the first tube body 13 is adapted to be in communication with the external cold source 200, and the first tube body 13 is adapted to continuously introduce the cooling medium from the external cold source 200 into the accommodating cavity 104;
a distal end of the second tube body 14 is in communication with the accommodating cavity 104, a proximal end of the second tube body 14 is adapted to be in communication with the external cold source 200, and the second tube body 14 is adapted to continuously guide the cooling medium after heat absorption from the accommodating cavity 104 to the external cold source 200.

In the present embodiment, the first tube body 13 and the second tube body 14 are disposed to form a circulating cooling loop between the cooling block body 11 and the external cold source 200, which not only prevents the generation of excess heat, avoids redundant heat from damaging surrounding blood vessels or nerves, but also can greatly improve the cooling efficiency of the cooling structure 103 for the first clamping body 101.

Optionally, referring to FIG. 3, a limiting groove (not shown in the figure) may be formed on the side of the first clamping body 101 away from the second clamping body 102 along the first direction to embed the first tube body 13 and the second tube body 14 in the limiting groove, which can not only reduce the damage risk of the first tube body 13 and the second tube body 14, but also help optimize the overall structure of the first clamping body 101, reduce the volume of the first clamping body 101, and facilitate the operator to perform more refined cutting or electrocoagulation operations during clinical surgery.

It can be understood that in the specific implementation process, technicians can adjust the specific structure and size of the side of the first clamping body 101 away from the second clamping body 102 along the first direction according to the actual shape and size of the cooling block body 11, the first sealing head 12, the first tube body 13 and the second tube body 14, which is not limited to the situation described in the present embodiment.

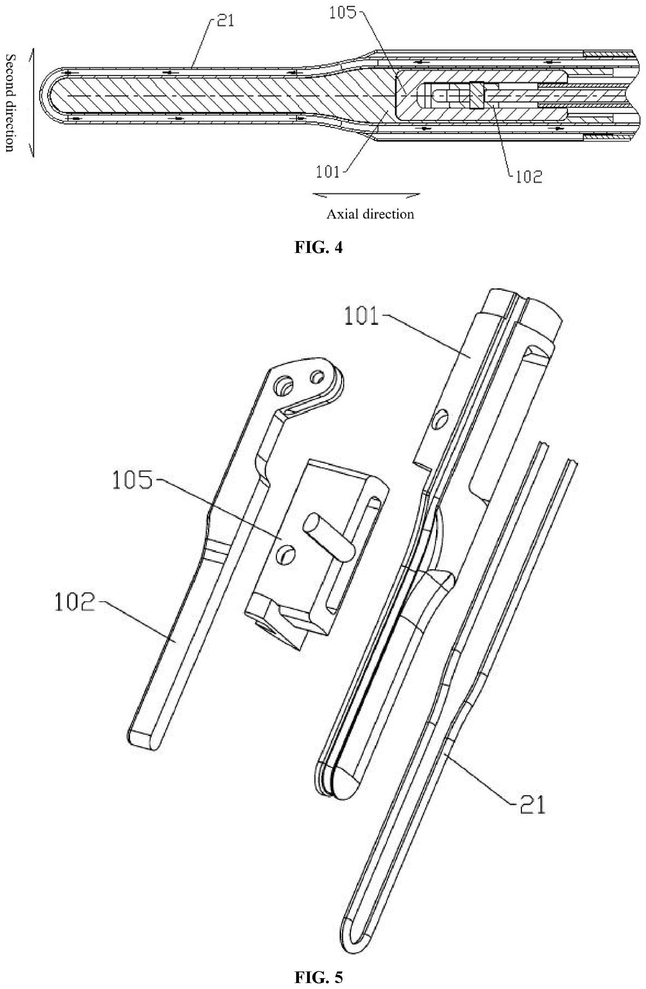

Referring to FIGS. 4 and 5 together, the following exemplarily provides a second type of energy-based surgery active cooling apparatus applied to monopolar and bipolar metal electrodes under laparoscopy, wherein the cooling structure 103 mainly includes a third tube body 21 which is of an integrally formed U-shaped structure and is independently disposed from the first clamping body 101, thereby forming an "independent U-shaped cooling structure".

In some embodiments, referring to FIG. 1, the first clamping body 101 includes a forceps body 106 and a forceps tip 107; referring to FIG. 5, the cooling structure 103 includes a third tube body 21, which is disposed on the side of the first clamping body 101 away from the second clamping body 102 along the first direction; referring to FIG. 4 together, the third tube body 21 is a U-shaped structure and is bent to change direction at the distal end position of the forceps tip 107 of the first clamping body 101;
the third tube body 21 is of an integrally formed structure, an input end of the third tube body 21 is in communication with a cooling medium output port of the external cold source 200, an output end of the third tube body 21 is in communication with a cooling medium return port of the external cold source 200, and the third tube body 21 is adapted to circulate the cooling medium to cool the first clamping body 101.

In the present embodiment, the input end of the third tube body 21 is in communication with the cooling medium output port of the external cold source 200, and the output end of the third tube body 21 is in communication with the cooling medium return port of the external cold source 200, thereby forming a circulating cooling loop with the external cold source 200. During operation, the third tube body 21 can timely take away the heat on both sides of the first clamping body 101 along the first direction without generating excess heat, so that a low-temperature safety zone is formed within the entire circumferential area of the first clamping body 101, thereby effectively avoiding damage to surrounding blood vessels or nerves and greatly improving the safety of electrosurgery. On the other hand, the third tube body 21 is of an integrally formed U-shaped structure, which does not require additional heat dissipation components and sealing components, thereby greatly simplifying the cooling structure and reducing the process difficulty.

Optionally, referring to FIG. 5, a U-shaped limiting groove (not shown in the figure) may be formed at the edge position of the first clamping body 101 to embed the third tube body 21 in the limiting groove, which can not only reduce the damage risk of the third tube body 21, but also help optimize the overall structure of the first clamping body 101, reduce the volume of the first clamping body 101, and facilitate the operator to perform more refined cutting or electrocoagulation operations during clinical surgery.

It can be understood that in the specific implementation process, technicians can adjust the specific structure and size of the limiting groove on the first clamping body 101 according to the actual shape and size of the third tube body 21, which is not limited to the situation described in the present embodiment.

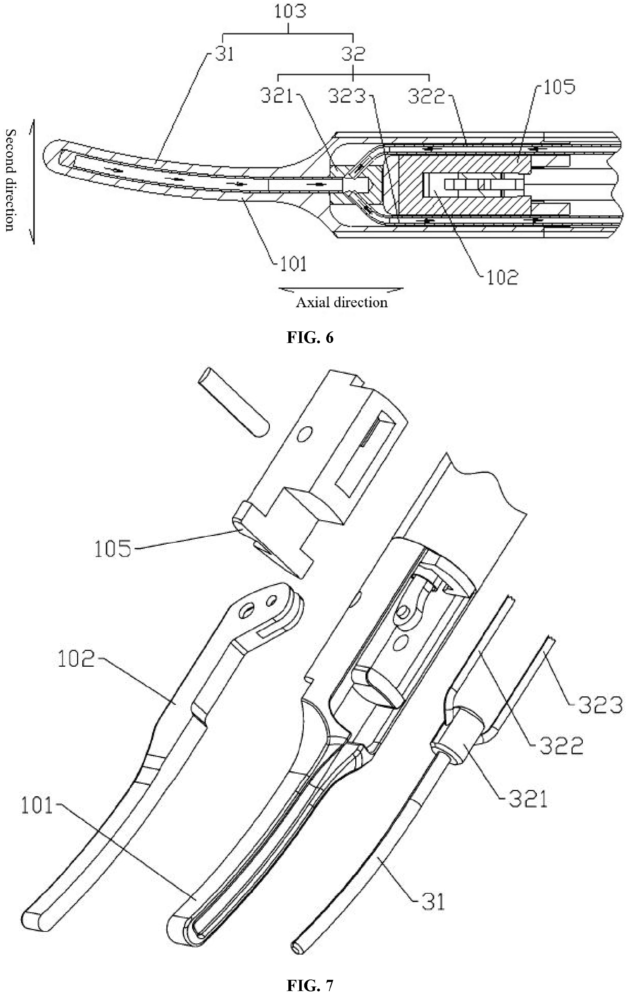

Referring to FIGS. 6 and 7 together, the following exemplarily provides a third type of energy-based surgery active cooling apparatus applied to monopolar and bipolar metal electrodes under laparoscopy, wherein the cooling structure 103 mainly includes a fourth tube body 31 and a circulating cooling assembly 32 which are both independently disposed from the first clamping body 101. Wherein, the circulating cooling assembly 32 mainly includes a communicating block 321, a fifth tube body 322 and a sixth tube body 323, thereby forming an "independent three-way block - independent triple-tube body cooling structure".

In some embodiments, referring to FIG. 1, the first clamping body 101 includes a forceps body 106 and a forceps tip 107; referring to FIG. 6, the cooling structure 103 includes a fourth tube body 31 and a circulating cooling assembly 32, which are disposed on the side of the first clamping body 101 away from the second clamping body 102 along the first direction;
the fourth tube body 31 is disposed at the end of the first clamping body 101 close to the forceps tip 107 along the axial direction, and the fourth tube body 31 is adapted to accommodate the cooling medium to cool and reduce the temperature of the first clamping body 101;
the circulating cooling assembly 32 is disposed at the end of the first clamping body 101 close to the forceps body 106 along the axial direction, a distal end of the circulating cooling assembly 32 is in communication with the fourth tube body 31, a proximal end of the circulating cooling assembly 32 is adapted to be in communication with the external cold source 200, and the circulating cooling assembly 32 is adapted to continuously cool the cooling medium in the fourth tube body 31.

It should be noted that referring to FIGS. 6 and 7 together, the fourth tube body 31 is embedded in the side of the first clamping body 101 away from the second clamping body 102 along the first direction, and the fourth tube body 31 is disposed at the end of the first clamping body 101 close to the forceps tip 107 along the axial direction, so as to cool and reduce the temperature of the first clamping body 101. The distal end of the fourth tube body 31 is closed, the proximal end of the fourth tube body 31 is in communication with the circulating cooling assembly 32, and the heat transfer between the fourth tube body 31 and the circulating cooling assembly 32 is mainly in the form of heat conduction between high-temperature cooling medium and low-temperature cooling medium, in addition to at least partial flow circulation of the cooling medium. During operation, a circulating loop is formed between the circulating cooling assembly 32 and the external cold source 200, and at the same time, heat is transferred between the circulating cooling assembly 32 and the fourth tube body 31, so as to conduct the heat on the first clamping body 101 to the external cold source 200, thereby realizing cooling and temperature reduction of the first clamping body 101.

It is worth noting that the fourth tube body 31 is disposed at the end of the first clamping body 101 close to the forceps tip 107 along the axial direction, which not only can realize cooling and temperature reduction of the first clamping body 101, but also can reduce the thickness along the first direction and the width along the second direction of the forceps tip 107 position of the first clamping body 101, thereby reducing the volume of the forceps tip 107. On the premise of ensuring the basic clamping and cutting functions as well as cooling efficiency, the first clamping body 101 can be more miniaturized, so as to avoid the forceps head touching nerves or blood vessels in a complex and narrow surgical space, avoid heat damage risks, and greatly improve the safety of electrosurgery.

In some embodiments, referring to FIG. 6, the circulating cooling assembly 32 includes a communicating block 321, a fifth tube body 322 and a sixth tube body 323, wherein the communicating block 321 is a three-way structure, and the communicating block 321 is adapted to simultaneously communicate the fourth tube body 31, the fifth tube body 322 and the sixth tube body 323;
both the fifth tube body 322 and the sixth tube body 323 are disposed at the proximal end of the first clamping body 101; the distal end of the fifth tube body 322 is in communication with the communicating block 321, the proximal end of the fifth tube body 322 is adapted to be in communication with the cooling medium output port of the external cold source 200; the distal end of the sixth tube body 323 is in communication with the communicating block 321, and the proximal end of the sixth tube body 323 is adapted to be in communication with the cooling medium return port of the external cold source 200.

In the present embodiment, the communicating block 321 is disposed to simultaneously communicate the fourth tube body 31, the fifth tube body 322 and the sixth tube body 323, so that the heat in the fourth tube body 31 can be conducted into the communicating block 321, and continuous circulating cooling is performed through the fifth tube body 322 and the sixth tube body 323, so as to conduct the heat in the fourth tube body 31 to the external cold source 200. This allows the fourth tube body 31 to timely take away the heat of the first clamping body 101 without generating excess heat, thereby effectively avoiding damage to surrounding blood vessels or nerves, and improving the safety of electrosurgery. At the same time, the first clamping body 101 can be more miniaturized, so as to avoid the forceps head touching nerves or blood vessels in a complex and narrow surgical space, avoid heat damage risks, and greatly improve the safety of electrosurgery.

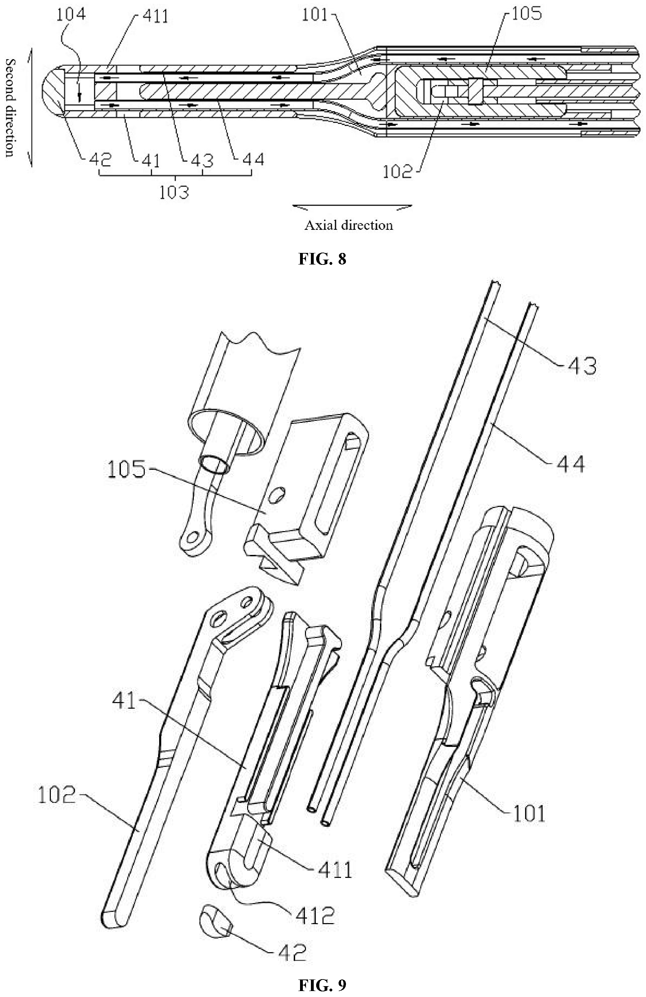

Referring to FIGS. 8 and 9 together, the following exemplarily provides a fourth type of energy-based surgery active cooling apparatus applied to monopolar and bipolar metal electrodes under laparoscopy, wherein the cooling structure 103 mainly includes a first plate body 41, a second sealing head 42, a seventh tube body 43 and an eighth tube body 44, the first plate body 41, the seventh tube body 43 and the eighth tube body 44 are all independently disposed relative to the first clamping body 101, and a cooling portion 411 is formed on a side of the first plate body 41 away from the second clamping body 102 along the first direction, thereby forming an "independent plate body and its cooling portion - independent double-tube body cooling structure".

In some embodiments, referring to FIG. 1, the first clamping body 101 includes a forceps body 106 and a forceps tip 107; referring to FIG. 9, the cooling structure 103 includes a first plate body 41 and a second sealing head 42;
the first plate body 41 is fixedly disposed on a side of the first clamping body 101 close to the second clamping body 102 along the first direction, and the first plate body 41 is adapted to abut against the second clamping body 102 to realize the clamping function; a cooling portion 411 is formed on the side of the first plate body 41 away from the second clamping body 102 along the first direction, the cooling portion 411 is disposed at an end of the first plate body 41 close to the forceps tip 107 along the axial direction, and the cooling portion 411 and the first plate body 41 are of an integrally formed structure;
referring to FIG. 9, a second accommodating groove 412 is formed on the cooling portion 411; referring to FIG. 8 together, the second sealing head 42 is adapted to sealing the second accommodating groove 412 to close the second accommodating groove 412 and form the accommodating cavity 104.

It should be noted that, the cooling portion 411 and the first plate body 41 in the present embodiment are of an integrally formed structure. Compared with the "independent cooling block - independent double-tube body cooling structure", in addition to the existing beneficial effects of the "independent cooling block - independent double-tube body cooling structure", it can also disassemble the part to be cooled of the first clamping body 101 into a single component to be directly connected to the waterway, which avoids the need to arrange an independent cooling block, thereby further improving the cooling and heat dissipation efficiency.

In some embodiments, referring to FIG. 9, the cooling structure 103 further includes a seventh tube body 43 and an eighth tube body 44, both of which are disposed on the side of the first clamping body 101 away from the second clamping body 102 along the first direction;
referring to FIG. 8 together, a distal end of the seventh tube body 43 is in communication with the accommodating cavity 104, a proximal end of the seventh tube body 43 is adapted to be in communication with the external cold source 200, and the seventh tube body 43 is adapted to continuously introduce the cooling medium from the external cold source 200 into the accommodating cavity 104;
a distal end of the eighth tube body 44 is in communication with the accommodating cavity 104, a proximal end of the eighth tube body 44 is adapted to be in communication with the external cold source 200, and the eighth tube body 44 is adapted to continuously guide the cooling medium after heat absorption from the accommodating cavity 104 to the external cold source 200.

In the present embodiment, the seventh tube body 43 and the eighth tube body 44 are disposed to form a circulating cooling loop between the cooling portion 411 of the first plate body 41 and the external cold source 200. This not only prevents the generation of excess heat, avoids redundant heat from damaging surrounding blood vessels or nerves, but also can greatly improve the cooling efficiency of the cooling structure 103 for the first plate body 41.

Optionally, referring to FIG. 9, two first limiting grooves (not shown in the figure) can be formed on the side of the first plate body 41 away from the second clamping body 102 along the first direction, and two second limiting grooves (not shown in the figure) can be respectively formed on both sides of the first clamping body 101 along the second direction, so as to embed the seventh tube body 43 and the eighth tube body 44 in the first limiting grooves and the second limiting grooves, which can not only reduce the damage risk of the seventh tube body 43 and the eighth tube body 44, but also help optimize the overall structure of the first plate body 41 and the first clamping body 101, reduce the volume of the first plate body 41 and the first clamping body 101, and facilitate the operator to perform more refined cutting or electrocoagulation operations during clinical surgery.

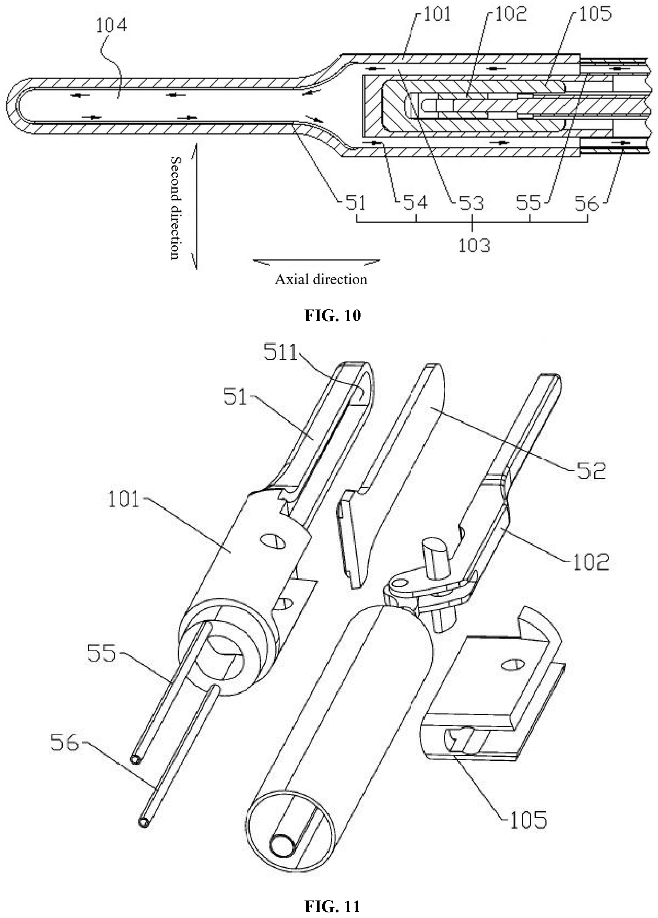

Referring to FIGS. 10 and 11 together, the following exemplarily provides a fifth type of energy-based surgery active cooling apparatus applied to monopolar and bipolar metal electrodes under laparoscopy, wherein the cooling structure 103 mainly includes a housing portion 51, a second plate body 52, a first through hole 53, a second through hole 54, a ninth tube body 55 and a tenth tube body 56. Wherein, the housing portion 51 and the first clamping body 101 are integrally formed, the first through hole 53 and the second through hole 54 are formed on the first clamping body 101, and the second plate body 52, the ninth tube body 55 and the tenth tube body 56 are all independently disposed relative to the first clamping body 101, thereby forming an "integral cooling groove - double deep holes - independent double- tube body cooling structure".

In some embodiments, referring to FIG. 1 again, the first clamping body 101 includes a forceps body 106 and a forceps tip 107; referring to FIG. 11, the cooling structure 103 includes a housing portion 51, which is disposed at the end of the first clamping body 101 close to the forceps tip 107 along the axial direction, and the housing portion 51 and the first clamping body 101 are integrally formed;
a third accommodating groove 511 is formed on a side of the housing portion 51 close to the second clamping body 102 along the first direction; the cooling structure 103 further includes a second plate body 52, which is fixedly disposed on the side of the first clamping body 101 close to the second clamping body 102 along the first direction, and the second plate body 52 is adapted to cover the third accommodating groove 511 to close the third accommodating groove 511 and form the accommodating cavity 104.

It should be noted that in the present embodiment, the housing portion 51 and the second plate body 52 are disposed to enclose and form a closed accommodating cavity 104 at the forceps tip 107 position of the first clamping body 101. Compared with any of the previous four types of energy-based surgery active cooling apparatuses applied to monopolar and bipolar metal electrodes under laparoscopy, the accommodating cavity 104 in the present embodiment has a larger volume and can accommodate more cooling medium, which greatly increases the cooling area for the first clamping body 101 and is conducive to improving the cooling efficiency of the first clamping body 101.

In some embodiments, referring to FIG. 10, a first through hole 53 and a second through hole 54 are formed at the proximal end of the first clamping body 101, and the first through hole 53 and the second through hole 54 respectively extend axially toward the forceps tip 107 position and are both in communication with the third accommodating groove 511;
referring to FIG. 11 together, the cooling structure 103 further includes a ninth tube body 55 and a tenth tube body 56, both of which are disposed at the end of the first clamping body 101 away from the forceps tip 107 along the axial direction;
a distal end of the ninth tube body 55 is in communication with the first through hole 53, a proximal end of the ninth tube body 55 is adapted to be in communication with the external cold source 200, and the ninth tube body 55 is adapted to continuously introduce the cooling medium from the external cold source 200 into the accommodating cavity 104 via the first through hole 53;
a distal end of the tenth tube body 56 is in communication with the second through hole 54, a proximal end of the tenth tube body 56 is adapted to be in communication with the external cold source 200, and the tenth tube body 56 is adapted to continuously guide the cooling medium after heat absorption from the accommodating cavity 104 to the external cold source 200 via the second through hole 54.

It should be noted that the first through hole 53 and the second through hole 54 can be formed on the first clamping body 101 by deep hole processing, so that both the first through hole 53 and the second through hole 54 are axially in communication with the third accommodating groove 511. The second plate body 52 can be welded and fixed to the side of the first clamping body 101 close to the second clamping body 102 along the first direction, so as to cover the third accommodating groove 511 and form a closed accommodating cavity 104.

In the present embodiment, the first through hole 53 and the ninth tube body 55, as well as the second through hole 54 and the tenth tube body 56 are disposed to form a circulating cooling loop between the accommodating cavity 104, enclosed by the housing portion 51 and the second plate body 52, and the external cold source 200, which not only prevents the generation of excess heat, avoids redundant heat from damaging surrounding blood vessels or nerves, but also can greatly improve the cooling efficiency of the cooling structure 103 for the first clamping body 101.

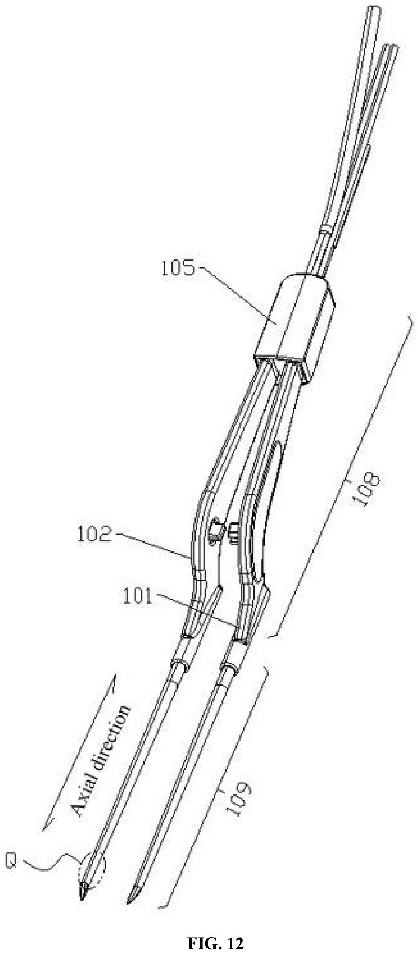

Referring to FIGS. 12, 13 and 15 together, the following exemplarily provides a first type of energy-based surgery active cooling apparatus applied to high-frequency bipolar tweezer, wherein the cooling structure 103 is disposed on any one of the first clamping body 101 and the second clamping body 102, thereby forming a "single-side tweezer body cooling structure"; the following description will be given by taking the first clamping body 101 as an example.

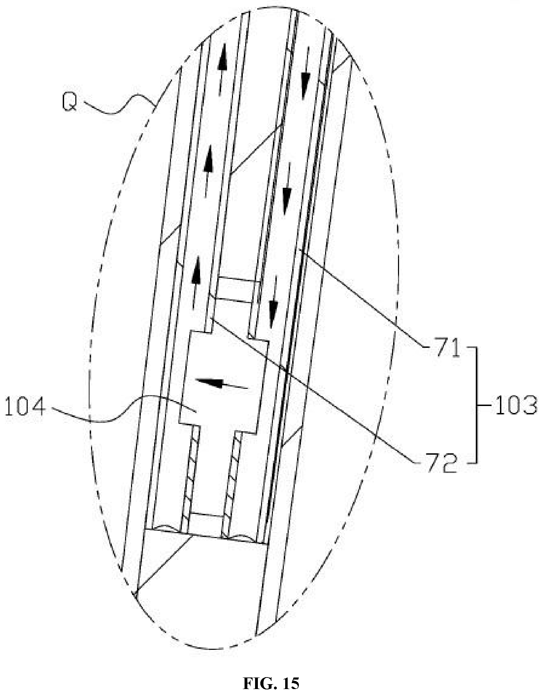

It should be noted that FIG. 15 shows the accommodating cavity 104 formed at the tweezer tip 109 position of the second clamping body 102, and the accommodating cavity formed at the tweezer tip 109 position of the first clamping body 101 can refer to the accommodating cavity 104 formed at the tweezer tip 109 position of the second clamping body 102 as shown in FIG. 15.

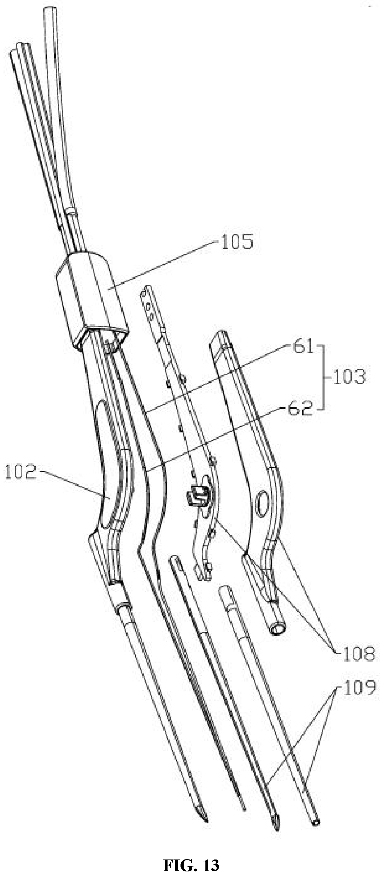

In some embodiments, referring to FIG. 12, the first clamping body 101 includes a tweezer body 108 and a tweezer tip 109; referring to FIG. 15 together, the tip position of the tweezer tip 109 of the first clamping body 101 is hollow inside and to form the accommodating cavity 104;
referring to FIG. 13, the cooling structure 103 includes an eleventh tube body 61 and a twelfth tube body 62, which are respectively disposed on both sides of the tweezer body 108 of the first clamping body 101 along the second direction and extend axially to the tip position of the tweezer tip 109 of the first clamping body 101;
a distal end of the eleventh tube body 61 is in communication with the accommodating cavity 104, a proximal end of the eleventh tube body 61 is in communication with the cooling medium output port of the external cold source 200, and the eleventh tube body 61 is adapted to continuously introduce the cooling medium from the external cold source 200 into the accommodating cavity 104; a distal end of the twelfth tube body 62 is in communication with the accommodating cavity 104, a proximal end of the twelfth tube body 62 is in communication with the cooling medium return port of the external cold source 200, and the twelfth tube body 62 is adapted to continuously guide the cooling medium after heat absorption from the accommodating cavity 104 to the external cold source 200.

Optionally, in the manufacturing process, the eleventh tube body 61 and the twelfth tube body 62 can be embedded in the tweezer body 108 and the tweezer tip 109 of the first clamping body 101, the distal ends of the eleventh tube body 61 and the twelfth tube body 62 are simultaneously in communication with the accommodating cavity 104 at the tip position of the tweezer tip 109 of the first clamping body 101, and the proximal ends of the eleventh tube body 61 and the twelfth tube body 62 are in communication with the external cold source 200, so that a circulating cooling loop is formed between the accommodating cavity 104 at the tip position of the tweezer tip 109 of the first clamping body 101 and the external cold source 200.

In the present embodiment, the eleventh tube body 61 and the twelfth tube body 62 are disposed to form a circulating cooling loop between the accommodating cavity 104 at the tip position of the tweezer tip 109 of the first clamping body 101 and the external cold source 200. During operation, the cooling structure 103 and the cooling medium therein can timely take away the heat at the tip of the tweezer tip 109 without generating excess heat, so that a low-temperature safety zone is formed within the entire circumferential area of the first clamping body 101 and/or the second clamping body 102. This facilitates the operator or physician to selectively use the first clamping body 101 as a fulcrum away from nerves or blood vessels in the actual clinical surgical process, effectively avoids damage to surrounding blood vessels or nerves caused by redundant heat generated during electrocoagulation or electrocision due to heating of both clamping bodies, and greatly improves the safety of electrosurgery. At the same time, it eliminates the heat risk of the heat-generating surface, and avoids the need to arrange another set of heat dissipation structures in the clamping body for synchronous heat conduction, which not only improves the heat dissipation and cooling efficiency of the clamping body and enhances the safety in the clinical surgical process, but also simplifies the heat dissipation structure of the clamping body and reduces the process difficulty of the energy-based surgery active cooling apparatus.

It should be noted that the principle of the "single-side tweezer body cooling structure" of the second clamping body 102 is the same as that of the first clamping body 101, and will not be repeated here.

Referring to FIGS. 12 to 15 together, the following exemplarily provides a second type of energy-based surgery active cooling apparatus applied to high-frequency bipolar tweezer, wherein the cooling structure 103 is disposed on both the first clamping body 101 and the second clamping body 102, thereby forming a "double-side tweezer body cooling structure". The cooling structure 103 of the first clamping body 101 has been given in the above embodiments, and will not be repeated here.

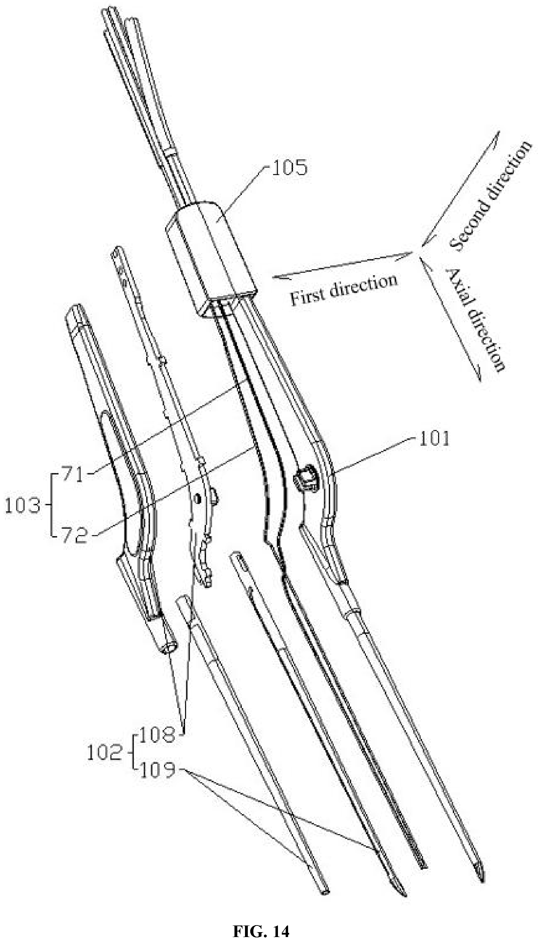

In some embodiments, referring to FIG. 12, the second clamping body 102 includes a tweezer body 108 and a tweezer tip 109; referring to FIG. 15, the tip position of the tweezer tip 109 of the second clamping body 102 is hollow inside and to form the accommodating cavity 104;
referring to FIG. 14, the cooling structure 103 includes a thirteenth tube body 71 and a fourteenth tube body 72, which are respectively disposed on both sides of the tweezer body 108 of the second clamping body 102 along the second direction and extend axially to the tip position of the tweezer tip 109 of the second clamping body 102;
a distal end of the thirteenth tube body 71 is in communication with the accommodating cavity 104, a proximal end of the thirteenth tube body 71 is in communication with the cooling medium output port of the external cold source 200, and the thirteenth tube body 71 is adapted to continuously introduce the cooling medium from the external cold source 200 into the accommodating cavity 104; a distal end of the fourteenth tube body 72 is in communication with the accommodating cavity 104, a proximal end of the fourteenth tube body 72 is in communication with the cooling medium return port of the external cold source 200, and the fourteenth tube body 72 is adapted to continuously guide the cooling medium after heat absorption from the accommodating cavity 104 to the external cold source 200.

It should be noted that compared with the above-mentioned "single-side tweezer body cooling structure", during operation, the cooling structure 103 of the "double-side tweezer body cooling structure" in the present embodiment and the cooling medium therein can timely take away the heat at the tips of the tweezer tips 109 of both clamping bodies without generating excess heat, so that a low-temperature safety zone is formed within the entire circumferential area of the first clamping body 101 and the second clamping body 102, thereby further improving the cooling and heat dissipation efficiency.

In some embodiments, the energy-based surgery active cooling apparatus further includes a connecting block 105, which is disposed between the first clamping body 101 and the second clamping body 102, and the connecting block 105 is adapted to connect the proximal end of the first clamping body 101 with the proximal end of the second clamping body 102.

It should be noted that referring to FIGS. 1 to 11, in the energy-based surgery active cooling apparatus applied to monopolar and bipolar metal electrodes under laparoscopy, the connecting block 105 is adapted to rotatably connect the first clamping body 101 and the second clamping body 102 to realize the basic clamping and opening functions. Referring to FIGS. 12 to 14, in the energy-based surgery active cooling apparatus applied to high-frequency bipolar tweezer, the connecting block 105 is adapted to fixedly connect the first clamping body 101 and the second clamping body 102 to realize the basic tweezer tip clamping function. Those skilled in the art can configure the specific structure of the connecting block 105 according to different application scenarios of the energy-based surgery active cooling apparatus, which is not limited to the situations described in FIGS. 1 to 14 of the above embodiments.

According to the embodiments of the present application, in another aspect, referring to FIGS. 1 to 17, an energy-based surgery active cooling system is further provided, including: an external cold source 200 and the energy-based surgery active cooling apparatus as described above, wherein the external cold source 200 is adapted to supply the cooling medium to the cooling structure 103.

The energy-based surgery active cooling system in the present solution includes the above-mentioned energy-based surgery active cooling apparatus, therefore, the energy-based surgery active cooling system in the present solution includes all the beneficial effects of the above-mentioned energy-based surgery active cooling apparatus.

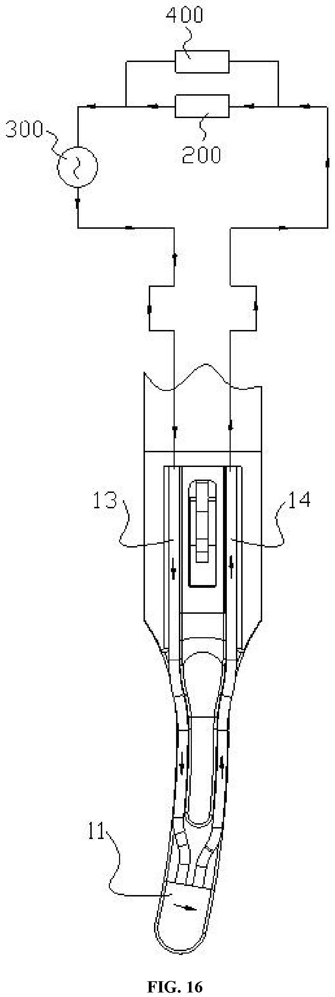

It should be noted that FIG. 16 shows a working principle diagram of an energy-based surgery active cooling system applied to monopolar and bipolar metal electrodes under laparoscopy. For better explanation and understanding, the present embodiment will be described in combination with the above first type of energy-based surgery active cooling apparatus applied to bipolar metal electrodes under laparoscopy. The distal ends of the first tube body 13 and the second tube body 14 of the energy-based surgery active cooling apparatus are respectively in communication with the accommodating cavity 104, the proximal end of the first tube body 13 can be in communication with the cooling medium output port of the external cold source 200, and the proximal end of the second tube body 14 can be in communication with the cooling medium return port of the external cold source 200, so as to continuously cool the heat-generating parts at the distal ends of the first clamping body 101 and/or the second clamping body 102 in a circulating manner without generating excess heat, thereby effectively avoiding damage to surrounding blood vessels or nerves caused by redundant heat generated during electrocoagulation or electrocision due to heating of the clamping bodies, and greatly improving the safety of electrosurgery. The working principles of the other energy-based surgery active cooling apparatuses applied to monopolar and bipolar metal electrodes under laparoscopy in this energy-based surgery active cooling system are the same as that of the first type, and will not be repeated here.

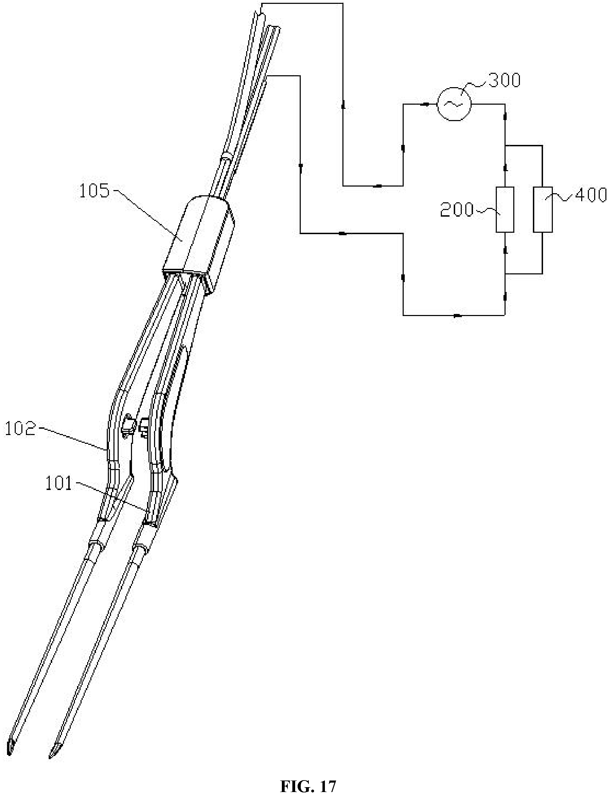

It should be noted that FIG. 17 shows a working principle diagram of an energy-based surgery active cooling system applied to high-frequency bipolar tweezer. The eleventh tube body 61 and the twelfth tube body 62 (and/or the thirteenth tube body 71 and the fourteenth tube body 72) of the energy-based surgery active cooling apparatus form a circulating cooling loop between the accommodating cavity 104 at the tip position of the tweezer tip 109 of the first clamping body 101 (and/or the second clamping body 102) and the external cold source 200, so as to continuously cool the heat-generating parts at the tips of the tweezer tips 109 of the first clamping body 101 and/or the second clamping body 102 in a circulating manner without generating excess heat, thereby effectively avoiding damage to surrounding blood vessels or nerves caused by redundant heat generated during electrocoagulation or electrocision due to heating of the clamping bodies, and greatly improving the safety of electrosurgery.

In some embodiments, the energy-based surgery active cooling system further includes a pumping device 300, which is adapted to provide power for the circulation of the cooling medium between the cooling structure 103 and the external cold source 200.

Optionally, the pumping device 300 can be a peristaltic pump.

In the present embodiment, the energy-based surgery active cooling system is provided with a pumping device 300 to provide power for the circulation of the cooling medium between the cooling structure 103 and the external cold source 200. During operation, the pumping device 300 can be used to adjust the circulation flow rate and flow volume of the cooling medium, thereby adjusting the cooling and temperature reduction effect of the cooling structure 103 for the clamping body.

In some embodiments, the energy-based surgery active cooling system further includes a refrigeration device 400 adapted to cool the cooling medium.

In the present embodiment, the energy-based surgery active cooling system is provided with a refrigeration device 400 to cool the cooling medium, thereby increasing the temperature difference between the heat-generating parts of the clamping body and the cooling medium, and improving the cooling and temperature reduction efficiency of the cooling structure 103 for the clamping body.

Although embodiments of the present application have been described in conjunction with the accompanying drawings, those skilled in the art may make modifications and variations without departing from the spirit and scope of the present application, and such modifications and variations fall within the scope defined by the appended claims.

## Claims

1. An energy-based surgery active cooling apparatus, **characterized by** comprising:
a first clamping body (101);
a second clamping body (102) connected to the first clamping body (101), wherein a distal end of the second clamping body (102) is adapted to abut against a distal end of the first clamping body (101);
a cooling structure (103) disposed on at least one of the first clamping body (101) and the second clamping body (102); wherein the cooling structure (103) is hollow inside and to form an accommodating cavity (104), which is adapted to accommodating a cooling medium; and
the accommodating cavity (104) is in communication with an external cold source (200), and the accommodating cavity (104) is adapted to circulate the cooling medium with the external cold source (200), so as to conduct heat on the first clamping body (101) and/or the second clamping body (102) from the distal tip to the external cold source (200).

2. The energy-based surgery active cooling apparatus according to claim 1, **characterized in that** the first clamping body (101) comprises a forceps body (106) and a forceps tip (107); the cooling structure (103) comprises a cooling block body (11) and a first sealing head (12);
the cooling block body (11) is independently disposed at a tip position of the forceps tip (107) of the first clamping body (101), the cooling block body (11) is disposed on a side of the first clamping body (101) away from the second clamping body (102) along a first direction, and the cooling block body (11) is attached to the side of the first clamping body (101) away from the second clamping body (102) along the first direction; and
a first accommodating groove (111) is formed on the cooling block body (11); the first sealing head (12) is adapted to seal the first accommodating groove (111) to close the first accommodating groove (111) and form the accommodating cavity (104).

3. The energy-based surgery active cooling apparatus according to claim 2, **characterized in that** the cooling structure (103) further comprises a first tube body (13) and a second tube body (14), both of which are disposed on the side of the first clamping body (101) away from the second clamping body (102) along the first direction;
a distal end of the first tube body (13) is in communication with the accommodating cavity (104), a proximal end of the first tube body (13) is adapted to be in communication with the external cold source (200), and the first tube body (13) is adapted to continuously introduce the cooling medium from the external cold source (200) into the accommodating cavity (104); and
a distal end of the second tube body (14) is in communication with the accommodating cavity (104), a proximal end of the second tube body (14) is adapted to be in communication with the external cold source (200), and the second tube body (14) is adapted to continuously guide the cooling medium after heat absorption from the accommodating cavity (104) to the external cold source (200).

4. The energy-based surgery active cooling apparatus according to claim 1, **characterized in that** the first clamping body (101) comprises a forceps body (106) and a forceps tip (107); the cooling structure (103) comprises a third tube body (21) , which is disposed on a side of the first clamping body (101) away from the second clamping body (102) along a first direction; the third tube body (21) is of a U-shaped structure and is bent and redirected at a distal end position of the forceps tip (107) of the first clamping body (101); and
the third tube body (21) is of an integrally formed structure, an input end of the third tube body (21) is in communication with a cooling medium output port of the external cold source (200), an output end of the third tube body (21) is in communication with a cooling medium return port of the external cold source (200), and the third tube body (21) is adapted to circulate the cooling medium to cool the first clamping body (101).

5. The energy-based surgery active cooling apparatus according to claim 1, **characterized in that** the first clamping body (101) comprises a forceps body (106) and a forceps tip (107); the cooling structure (103) comprises a fourth tube body (31) and a circulating cooling assembly (32), both of which are disposed on a side of the first clamping body (101) away from the second clamping body (102) along a first direction;
the fourth tube body (31) is disposed at an end of the first clamping body (101) close to the forceps tip (107) along an axial direction, and the fourth tube body (31) is adapted to accommodate the cooling medium to cool and reduce the temperature of the first clamping body (101); and
the circulating cooling assembly (32) is disposed at an end of the first clamping body (101) close to the forceps body (106) along the axial direction, a distal end of the circulating cooling assembly (32) is in communication with the fourth tube body (31), a proximal end of the circulating cooling assembly (32) is adapted to being in communication with the external cold source (200), and the circulating cooling assembly (32) is adapted to continuously cooling the cooling medium in the fourth tube body (31).

6. The energy-based surgery active cooling apparatus according to claim 5, **characterized in that** the circulating cooling assembly (32) comprises a communicating block (321), a fifth tube body (322) and a sixth tube body (323), and the communicating block (321) is adapted to simultaneously communicate the fourth tube body (31), the fifth tube body (322) and the sixth tube body (323); and
both the fifth tube body (322) and the sixth tube body (323) are disposed at a proximal end of the first clamping body (101); a distal end of the fifth tube body (322) is in communication with the communicating block (321), a proximal end of the fifth tube body (322) is adapted to be in communication with a cooling medium output port of the external cold source (200); a distal end of the sixth tube body (323) is in communication with the communicating block (321), and a proximal end of the sixth tube body (323) is adapted to be in communication with a cooling medium return port of the external cold source (200).

7. The energy-based surgery active cooling apparatus according to claim 1, **characterized in that** the first clamping body (101) comprises a forceps body (106) and a forceps tip (107); the cooling structure (103) includes a first plate body (41) and a second sealing head (42);
the first plate body (41) is fixedly disposed on a side of the first clamping body (101) close to the second clamping body (102) along a first direction, and the first plate body (41) is adapted to abut against the second clamping body (102); a cooling portion (411) is formed on a side of the first plate body (41) away from the second clamping body (102) along the first direction, the cooling portion (411) is disposed at an end of the first plate body (41) close to the forceps tip (107) along an axial direction, and the cooling portion (411) and the first plate body (41) are of an integrally formed structure; and
a second accommodating groove (412) is formed on the cooling portion (411); the second sealing head (42) is adapted to seal the second accommodating groove (412) to close the second accommodating groove (412) and form the accommodating cavity (104).

8. The energy-based surgery active cooling apparatus according to claim 7, **characterized in that** the cooling structure (103) further comprises a seventh tube body (43) and an eighth tube body (44), both of which are disposed on a side of the first clamping body (101) away from the second clamping body (102) along the first direction;
a distal end of the seventh tube body (43) is in communication with the accommodating cavity (104), a proximal end of the seventh tube body (43) is adapted to be in communication with the external cold source (200), and the seventh tube body (43) is adapted to continuously introduce the cooling medium from the external cold source (200) into the accommodating cavity (104); and
a distal end of the eighth tube body (44) is in communication with the accommodating cavity (104), a proximal end of the eighth tube body (44) is adapted to be in communication with the external cold source (200), and the eighth tube body (44) is adapted to continuously guide the cooling medium after heat absorption from the accommodating cavity (104) to the external cold source (200).

9. The energy-based surgery active cooling apparatus according to claim 1, **characterized in that** the first clamping body (101) comprises a forceps body (106) and a forceps tip (107); the cooling structure (103) comprises a housing portion (51), which is disposed at an end of the first clamping body (101) close to the forceps tip (107) along an axial direction, and the housing portion (51) and the first clamping body (101) are integrally formed; and
a third accommodating groove (511) is formed on a side of the housing portion (51) close to the second clamping body (102) along a first direction; the cooling structure (103) further comprises a second plate body (52) , which is fixedly disposed on a side of the first clamping body (101) close to the second clamping body (102) along the first direction, and the second plate body (52) is adapted to cover the third accommodating groove (511) to close the third accommodating groove (511) and form the accommodating cavity (104).

10. The energy-based surgery active cooling apparatus according to claim 9, **characterized in that** a first through hole (53) and a second through hole (54) are formed at a proximal end of the first clamping body (101), and the first through hole (53) and the second through hole (54) respectively extend axially toward the forceps tip (107) and are both in communication with the third accommodating groove (511);
the cooling structure (103) further comprises a ninth tube body (55) and a tenth tube body (56), both of which are disposed at an end of the first clamping body (101) away from the forceps tip (107) along the axial direction;
a distal end of the ninth tube body (55) is in communication with the first through hole (53), a proximal end of the ninth tube body (55) is adapted to be in communication with the external cold source (200), and the ninth tube body (55) is adapted to continuously introduce the cooling medium from the external cold source (200) into the accommodating cavity (104) via the first through hole (53); and
a distal end of the tenth tube body (56) is in communication with the second through hole (54), a proximal end of the tenth tube body (56) is adapted to be in communication with the external cold source (200), and the tenth tube body (56) is adapted to continuously guide the cooling medium after heat absorption from the accommodating cavity (104) to the external cold source (200) via the second through hole (54).

11. The energy-based surgery active cooling apparatus according to claim 1, **characterized in that** the first clamping body (101) comprises a tweezer body (108) and a tweezer tip (109); the tweezer tip (109) position of the first clamping body (101) is hollow inside and to form the accommodating cavity (104);
the cooling structure (103) comprises an eleventh tube body (61) and a twelfth tube body (62), which are respectively disposed on both sides of the tweezer body (108) of the first clamping body (101) along a second direction and extend axially to the tip position of the tweezer tip (109) of the first clamping body (101); and
a distal end of the eleventh tube body (61) is in communication with the accommodating cavity (104), a proximal end of the eleventh tube body (61) is in communication with a cooling medium output port of the external cold source (200), and the eleventh tube body (61) is adapted to continuously introduce the cooling medium from the external cold source (200) into the accommodating cavity (104); a distal end of the twelfth tube body (62) is in communication with the accommodating cavity (104), a proximal end of the twelfth tube body (62) is in communication with a cooling medium return port of the external cold source (200), and the twelfth tube body (62) is adapted to continuously guide the cooling medium after heat absorption from the accommodating cavity (104) to the external cold source (200).

12. The energy-based surgery active cooling apparatus according to claim 11, **characterized in that** the second clamping body (102) comprises a tweezer body (108) and a tweezer tip (109); the tweezer tip (109) position of the second clamping body (102) is hollow inside and to form the accommodating cavity (104);
the cooling structure (103) comprises a thirteenth tube body (71) and a fourteenth tube body (72), which are respectively disposed on both sides of the tweezer body (108) of the second clamping body (102) along the second direction and extend axially to the tip position of the tweezer tip (109) of the second clamping body (102); and
a distal end of the thirteenth tube body (71) is in communication with the accommodating cavity (104), a proximal end of the thirteenth tube body (71) is in communication with the cooling medium output port of the external cold source (200), and the thirteenth tube body (71) is adapted to continuously introduce the cooling medium from the external cold source (200) into the accommodating cavity (104); a distal end of the fourteenth tube body (72) is in communication with the accommodating cavity (104), a proximal end of the fourteenth tube body (72) is in communication with the cooling medium return port of the external cold source (200), and the fourteenth tube body (72) is adapted to continuously guide the cooling medium after heat absorption from the accommodating cavity (104) to the external cold source (200).

13. The energy-based surgery active cooling apparatus according to claim 1, **characterized in that** the energy-based surgery active cooling apparatus further comprises a connecting block (105), which is disposed between the first clamping body (101) and the second clamping body (102), and the connecting block (105) is adapted to connect a proximal end of the first clamping body (101) with a proximal end of the second clamping body (102).

14. An energy-based surgery active cooling system, **characterized by** comprising: an external cold source (200) and the energy-based surgery active cooling apparatus according to any one of claims 1-13, wherein the external cold source (200) is adapted to supply the cooling medium to the cooling structure (103).

15. The energy-based surgery active cooling system according to claim 14, **characterized in that** the energy-based surgery active cooling system further comprises a pumping device (300), which is adapted to provide power for the circulation of the cooling medium between the cooling structure (103) and the external cold source (200).

16. The energy-based surgery active cooling system according to claim 14, **characterized in that** the energy-based surgery active cooling system further comprises a refrigeration device (400), which is adapted to cool the cooling medium.
